# EUROPEAN PATENT APPLICATION

(11) **EP 2 090 223 A1**
(43) Date of publication of application: **19.08.2009**
(21) Application number: 08465003.5
(22) Date of filing: 15.02.2008
(51) Int. Cl.: A61B 3/135

(54) **Optical set for examining of objects and method for examining of objects using optical devices**

(71) Applicant: OPTOPOL Technology Spolka Akcyjna, 42-400 Zawiercie (PL)
(72) Inventor: Wojtkowski, Maciej, 87-851, Boniewo (PL); Wojdas, Pawel, 42-400, Zawiercie (PL); Szkulmowski, Maciej, 87-100 Torun (PL)
(74) Representative: Hudy, Ludwik

(57) **Abstract**

In an optical set for examining of objects comprising at least a slit lamp (140) having a body and comprising a positioning arrangement (130) for moving the slit lamp (140) with respect to an examined object (101), an optical arrangement and a projector (142) generating a beam of light, on the body (148) of the slit lamp (140) there is mounted, via a coupling arrangement (150), movably with respect to the body (148) of the slit lamp (140), a device for imaging objects using spectral optical coherence tomography (a SOCT device) (160), having at least part of an optical arrangement (163) comprising an object beam during examination of the object (101) by means of the SOCT device (160) situated between the optical arrangement (146) of the slit lamp (140) and the examined object (101).

## Description

The object of the invention is an optical set for examining of objects and a method for examining of objects using optical devices, in particular using a slit lamp and other devices for non-invasive, in vivo examination of anatomical objects.

One type of device for examining such objects are slit lamps, used in particular for examining transparent and semi-transparent objects, which are illuminated during the examination by a light beam formed by an illumination slit. The examined object, for example an eye of the examined person, has only that part under examination illuminated. A typical slit lamp consists of a stand for supporting the head of the examined person, a device for generating a light beam and an optical arrangement, which enables observation of the illuminated part of the eye. In addition, the slit lamp may comprise an additional system for illuminating a peripheral portion of the fragment of the eye which is seen by the light from the first illumination system, as described in the US patent No. 6,072,623 entitled *"Slit lamp microscope".*

In turn, from PCT application No. WO 2007/055668 entitled *"Portable slit lamp with built-in digital camera and illuminating diaphragm",* is known a slit lamp provided with a camera with a special illumination aperture, which gives light of a proper quality, thereby enabling good quality pictures to be taken.

Other known optical devices are those for imaging of objects using optical tomography, which is one of the techniques for imaging objects and their structures, based on analysis of a signal generated by interference of a reference light beam and an object light beam, which returns to the device after being scattered on an object and reflected from it. Devices for imaging objects and/or methods for imaging objects using optical tomography are known for their variety of embodiments, and many of them are provided with additional equipment. For example, from a PCT application No. WO 2007/148310 A2 entitled *"Apparatus for optical frequency domain tomography with adjusting system, adjusting system of apparatus for optical frequency domain tomography and method for adjusting apparatus for optical frequency domain tomography"* is known a device for optical frequency domain tomography with an adjusting system. The examination of an object is based on analyzing of a resultant beam generated by interference of a reference light beam and an object light beam, which is scattered on an object, partly reflected and returning from the object. The resultant beam is directed to a dispersion device, for example a diffraction grating, and is next registered by a detection device or a spectrum recorder, for example a matrix of photosensitive elements, which are usually installed in linear CCD cameras. The signal generated by the recorder, usually in a digital form, is transmitted to a calculating unit, and information on axial structure of the object is obtained by numerical calculations performed by the calculating unit, for example by a PC. In order to increase the accuracy of object imaging, the device for optical frequency domain tomography according to the above-mentioned publication is provided with an automatically-controlled adjusting system, which effects relative displacement of at least one photosensitive element of the detection device and a spectrum image of the resultant beam, until the optimal projection of the spectrum image on the elements of the photosensitive detection device is achieved.

Presently known optical devices do not allow thorough examination of transparent and semi-transparent objects, for example an eye.

The object of the invention is that in an optical set for examining objects comprising at least a slit lamp having a body and comprising a positioning arrangement for moving the slit lamp with respect to an examined object, an optical arrangement and a projector generating a beam of light, on the body of the slit lamp there is mounted, via a coupling arrangement, movably with respect to the body of the slit lamp, a device for imaging objects using spectral optical coherence tomography (a SOCT device), having at least part of an optical arrangement comprising an object beam during examination of the object by means of the SOCT device situated between the optical arrangement of the slit lamp and the examined object.

Preferably, an axis of the optical arrangement of the SOCT device, during examination of the object by means of the SOCT device, is situated approximately coaxially aligned with an axis of the optical arrangement of the slit lamp and an axis of the examined object.

It is advisable that a positioning arrangement of the optical arrangement of the slit lamp during examination of the object by means of the slit lamp is suitable for positioning the optical arrangement of the SOCT device during examination of the object by means of the SOCT device.

In a preferable embodiment, the positioning arrangement is common for the optical arrangement of the slit lamp during examination of the object by means of the slit lamp and for the optical arrangement of the SOCT device during examination of the object by means of the SOCT device.

Preferably, the coupling arrangement, which allows moving of the SOCT device from a neutral position to an operating position and back to the neutral position, comprises an adapter, which has a shape conforming to the elements of the casing of the slit lamp and is attached to the casing, and to which a pin is fixed, on which is mounted a rotatable sleeve bearing, which is mounted within the body of the SOCT device.

The object of the invention is also a method for examining of objects using a slit lamp, in which during examination of the object by means of the slit lamp, the examined object is illuminated by a linear or rectilinear beam of light generated by a device for generating a linear or rectilinear beam and observation of the object is carried out by means of the optical arrangement of the slit lamp, and for the period of examination of the object by means of the device for imaging of objects using spectral optical coherence tomography (a SOCT device), the head of the projector generating the linear or rectilinear beam of light is moved aside, and next the SOCT device is shifted with respect to the slit lamp to a position, in which the optical arrangement of the SOCT device is situated approximately coaxially aligned with the optical arrangement of the slit lamp, and next the beam of light generated by the source of light of the SOCT device is split into a reference beam and an object beam, which is directed to the examined object, and after its reflection from the object, an interference of the object beam occurs with the reference light beam reflected from the reference mirror, thereby creating a resultant beam, which is directed to a dispersion device, and next a spectrum from the resultant beam is recorded by the detection device as a signal transmitted to a calculating unit, and information about the axial structure of the examined object is obtained by numerical calculations.

The SOCT device can be moved manually with respect to the slit lamp from an operating or examination position to a neutral position and from the neutral position to the operating position, by acting with force on the elements of the SOCT device.

The invention will be presented by reference to embodiments shown in a drawing, on which:
Fig. 1 presents a set comprising a slit lamp and a device in a perspective view, during examination by the device for imaging of objects using spectral optical coherence tomography (SOCT).
Fig. 2 presents a set comprising a slit lamp and a device for imaging of objects using spectral optical coherence tomography in a side view, during examination by the device for imaging of objects using spectral optical coherence tomography.
Fig. 3 presents a set comprising a slit lamp and a device for imaging of objects using spectral optical coherence tomography in a perspective view, during examination by the slit lamp.
Fig. 4 presents a set comprising a slit lamp and a device for imaging of objects using spectral optical coherence tomography in a side view, during examination by the slit lamp.

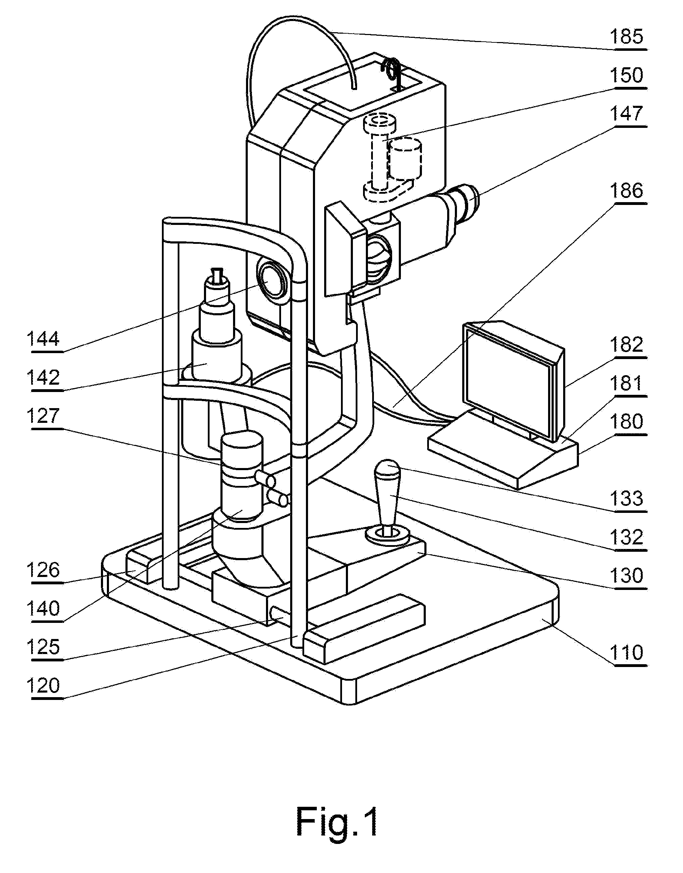

A set, to which the present invention can be applied, comprises a number of elements, of which the ones essential to understanding of the invention are shown in Figs. 1 - 4. Equivalent elements on all drawings are labeled by the same numerical references or references of which the first digit refers to the figure number. The basic elements of the set shown in the drawings are a slit lamp 140 with a base 110 and, mounted thereon, a device 160 for imaging objects using spectral optical coherence tomography, shortly called a SOCT device 160.

In the simplest embodiment, the SOCT device 160 comprises a light source generating a beam of light, which is split into two arms by a beam splitter. In the object arm, the beam of light penetrates the object 101 and is back-scattered on its internal structures and returns to the beam splitter. Simultaneously, the light in the reference arm is reflected from a stationary reference mirror and also returns to the beam splitter, from which a resultant beam is formed, which is next processed by a spectrometer. In the spectrometer, the resultant beam is directed to dispersion device, for example a diffraction grating, which splits the resultant beam into a spectrum of light, modulated by interference fringes, which is registered by a detection device. The electric signal generated by the detection device is transmitted to a calculating unit 181 powered by a power and control device 180. Information about the axial structure of the examined object is obtained by numerical calculations performed in the calculation unit 181, for example in the PC. The SOCT device 160 communicates with the calculation unit via a transmission line 185, which may further comprise power wires for the SOCT device 160. The image of the structure of the examined object 101 may be displayed on a monitor 182. Most of the above-mentioned devices and elements, necessary for operation of the SOCT device 160 are mounted within the body or the casing of the SOCT device 160.

In the set according to the invention, the SOCT device 160 is mounted on the slit lamp 140, which comprises a positioning device 130 for moving of the slit lamp with respect to the examined object 101, for example an eye of the examined person. The operating element of the positioning device is a manipulator 132, enabling displacement in horizontal direction (so-called horizontal movement) of the slit lamp 140 and the SOCT device 160 in a direction 135 parallel to the axis of the optical arrangement of the slit lamp 140, by means of guiding elements located in guides 126, as well as in a direction 136 transverse or laterally to the axis of the optical arrangement of the slit lamp 140 and of the SOCT device 160. The displacement in the lateral direction is effected during movement of the positioning device along the guide 125. The displacement in a vertical direction (so-called vertical movement) of the slit lamp 140 and of the SOCT device 160 is effected by a head 133 located on the manipulator 132. Horizontal movement in both directions may be utilized by means of a lever formed by the manipulator 132, having its second end 131 resting on the base 110, and by means of a head with elastic spring coupled with an arrangement 127 for vertical movement of the slit lamp. It is important that due to the fact that the SOCT device 160 is mounted on the slit lamp 140, the movement of both the slit lamp 140 and the SOCT device 160 may be effected by the same elements, namely the manipulator 132 and the head 133.

Instead of the mechanical devices for movement of the slit lamp 140 and the SOCT device 160, electronic devices can be used, having working elements enabling movement of the slit lamp 140 and the SOCT device 160 with respect to the examined object, for example a head of the examined object 101, which is placed on a moveable chin rest along guides 120 fixed to the base 110, and which can be supported by a forehead rest 122. During examination, the central axis 161 of the examined object 101 is at the same height 191 as the axis 162 of the optical arrangement 163, in particular the axis of the lens of the SOCT device 160, where in the presented embodiment the height is measured from the top surface of the base 110.

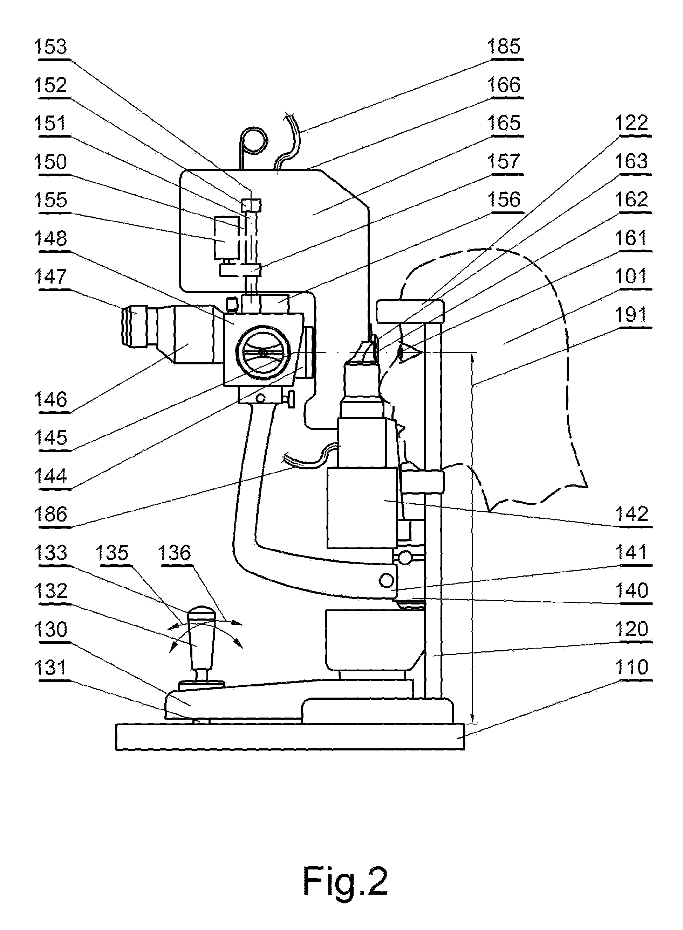

An optical arrangement 146 with binoculars 147 of the slit lamp 140, placed within a casing 148, is mounted on an extension arm 141 and is separated from the examined object 101. This creates space between the optical arrangement 146 of the slit lamp 140, in particular the lens 144 of the slit lamp 140, and the examined object 101. After the SOCT device 160 is moved from a neutral position to an operating position, at least part of the optical arrangement 163, comprising the object beam during examination of the object 101 using the SOCT device 160, is situated in the space between the optical arrangement 146, in particular the lens 144 of the slit lamp 140, forming a linear beam of light, and the examined object 101. Such configuration of the SOCT device is shown in Fig. 1 and 2. When using optical tomography, a projector 142, generating a linear beam of light having a rectangular or semi-rectangular cross section, is moved to a side position, such that it does not interrupt the examination. In turn, movement of the SOCT device 160 from a neutral position to an operating position is enabled by a coupling arrangement 150. In the embodiment shown in Figs. 1 and 2, the movement is effected by rotation of the SOCT device 160 around the axis 153 of the coupling arrangement 150. The term "optical arrangement 163 of the SOCT device 160" is to be understood as a part of the SOCT device 160 comprising at least arrangements or an arrangement for generating a beam of light, a light beam splitter, an object arm of the light beam, which penetrates the object 101 and is back-scattered on its internal structures, a reference arm of the light beam, a resulting beam arm, a spectrometer and a diffraction grating.

In another embodiment of the set comprising the slit lamp 140 and the SOCT device 160, it is possible to mount, on the body of the slit lamp 140, guides on which the SOCT device 160 can be moved to the examination position from the neutral or rest position and from the neutral position to the examination position.

The coupling or intermediary arrangement 150, which allows movement of the SOCT device 160 from the neutral position to the operating position and back to the neutral position, comprises, among other elements, an adapter 156, which is shaped to match the elements of the casing of the slit lamp and can be attached to it, for example by screws. The adapter 156 has a pin 151 fixed thereto, on which is mounted a rotatable sleeve bearing 152, which is mounted within the SOCT device 160, for example within the top part 166 of the body 165 of the SOCT device 160. Movement of the SOCT device 160 can be effected manually by the operator of the set, the force directed to the body 165 of the SOCT device 160. Alternatively, the movement can be effected by a driving arrangement 155 with a transmission gear 157. The driving arrangement 155 can be activated by means of the manipulator 132, for example by pressing the manipulator down. The body 165 presented in Fig. 1 and 2 has a shape of inverted letter "L", so that when the SOCT device 160 is rotated around the axis 153, the lower part of the body comprising the optical arrangement 163 of the SOCT device 160 does not interfere with the elements of the slit lamp 140 and therefore it can be moved to a spacing between the lens 145 of the slit lamp and the examined object 101. The SOCT device 160 may include blocking and/or latching devices, which prevent accidental displacement of the SOCT device 160 from the neutral or operating position.

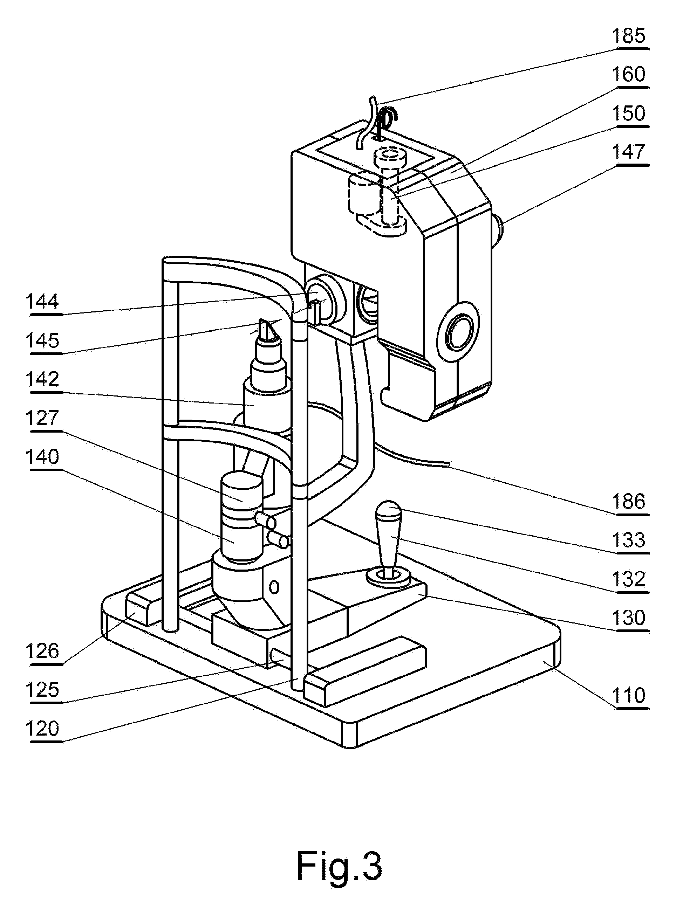

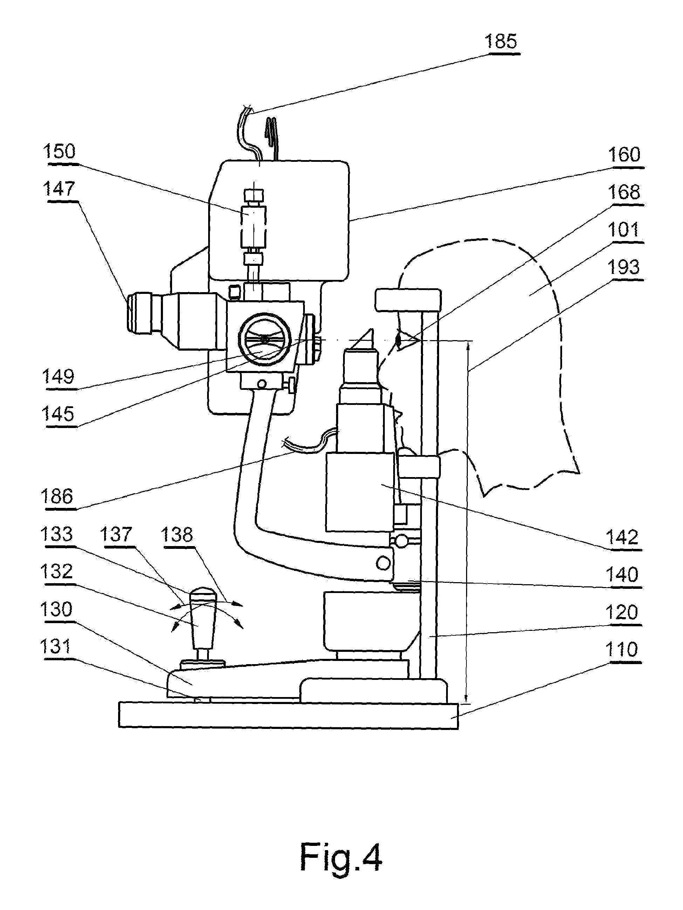

Fig. 3 and 4 present a set comprising the slit lamp 140 and the SOCT device 160 in a position in which examination of the object 101 is performed using the slit lamp 140, and the SOCT device is in a neutral or rest position, and its lower part of the body comprising the optical arrangement is located by the side of the slit lamp 140. The head of the projector 142 generating a linear beam of light is set in the axis 145 of the optical arrangement of the slit lamp 140 with a focusing arrangement 149, in particular in the axis of the lens 144. The axis 145 of the optical arrangement of the slit lamp, during examination of the object 101 using the slit lamp, is situated at a height 193, measured from the top surface of the base 110 to an axis 168 of the object 101, which is essentially the same as the height 191 shown in Fig. 2, at which the axis 162 of the optical arrangement 163 of the SOCT device, during examination of the object 101 using the SOCT device 160, is located. The head of the projector 142 generating the linear of rectilinear beam powered by a power and control device 180 via a transmission line 186, which may include wires for controlling the operation of the slit lamp 140, as well as wires transmitting signals from the controller 130, whose movement in directions 137, 138 or their combination effects displacement in horizontal direction, so-called horizontal movement, of the slit lamp 140 and the SOCT device 160.

The set comprising the slit lamp 140 and the SOCT device 160 enables thorough examination of objects without the need to displace the examined object 101, in particular examination of the front and/or back part of the human eye.

During examination of the object 101 using the slit lamp 140, the examined object is illuminated by the linear beam of light generated by the head of the projector 142 generating the linear beam of light and observation of the object is performed using the optical arrangement of the slit lamp. In turn, during examination of the object using the SOCT device 160, the projector 142 generating the linear or rectilinear beam of light is moved aside, and next the SOCT device 160 is moved with respect to the slit lamp 140 to a position, in which the optical arrangement of the SOCT device 160 is situated approximately coaxially aligned with the optical arrangement of the slit lamp 140, and next the beam of light generated by the source of light is split to a reference beam and an object beam, which is directed to the examined object 101, and after reflection from the object, interference with the reference beam of light reflected from the reference mirror is effected, thereby creating a resultant beam, which is directed to a dispersion device, and next the generated spectrum of the resultant beam is recorded by a detection device in form of a signal, which is transmitted to a calculating unit 181, and the information on axial structure of the examined object is obtained by means of numerical calculations performed in the calculating unit 181.

The invention has been shown with reference to selected embodiments. However, the embodiments shown are not to limit the invention. It is apparent that certain modifications can be introduced, which do not limit the object of the invention. The presented embodiments do not show all the possible applications of the invention.

## Claims

1. An optical set for examining of objects comprising at least a slit lamp having a body and comprising a positioning arrangement for moving the slit lamp with respect to an examined object, an optical arrangement and a projector generating a beam of light, **characterized in that,** on the body (148) of the slit lamp (140) is mounted, via a coupling arrangement (150), movable with respect to the body (148) of the slit lamp (140), a device for imaging objects using spectral optical coherence tomography (a SOCT device) (160), having at least an optical arrangement (163) comprising an object beam to be used during examination of the object (101) by means of the SOCT device (160) situated between the optical arrangement (146) of the slit lamp (140) and the examined object (101).

2. The optical set for examining of objects according to claim 1, **characterized in that** an axis of the optical arrangement of the SOCT device, during examination of the object (101) by means of the SOCT device (160), is situated approximately coaxially aligned with an axis of the optical arrangement of the slit lamp (140) and an axis of the examined object (101).

3. The optical set for examining of objects according to claim 1, **characterized in that** a positioning arrangement (130) of the optical arrangement of the slit lamp (140) during examination of the object (101) by means of the slit lamp (140) is suitable for positioning the optical arrangement (163) of the SOCT device (160) during examination of the object (101) by the SOCT device (160).

4. The optical set for examining of objects according to claim 1, **characterized in that** the positioning arrangement (130) is common for the optical arrangement of the slit lamp (140) during examination of the object (101) by means of the slit lamp (140) and for the optical arrangement (163) of the SOCT device (160) during examination of the object (101) by the SOCT device (160).

5. The optical set for examining of objects according to claim 1, **characterized in that** the coupling arrangement (150), which allows moving the SOCT device (160) from a neutral position to an operating position and back to the neutral position, comprises an adapter (156), which has a shape conforming to the elements of the casing of the slit lamp and is attached to the casing, and to which a pin (151) is fixed, on which is mounted a rotatable sleeve bearing (152), which is mounted within the body of the SOCT device (160).

6. A method for examining of objects using optical devices according to claim 1, **characterized in that** during examination of the object by means of the slit lamp, the examined object is illuminated by a linear or rectilinear beam of light generated by a device for generating a linear or rectilinear beam and observation of the object is carried out by means of the optical arrangement of the slit lamp, and for the period of examination of the object by means of the device for imaging of objects using spectral optical coherence tomography (a SOCT device), the head of the projector generating the linear or rectilinear beam of light is moved aside, and next the SOCT device is shifted with respect to the slit lamp to a position, in which the optical arrangement of the SOCT device is situated approximately coaxially aligned with the optical arrangement of the slit lamp, and next the beam of light generated by the source of light of the SOCT device is split into a reference beam and an object beam, which is directed to the examined object, and after its reflection from the object, an interference of the object beam occurs with the reference light beam reflected from the reference mirror, thereby creating a resultant beam, which is directed to a dispersion device, and next a spectrum from the resultant beam is recorded by the detection device as a signal transmitted to a calculating unit, and information about the axial structure of the examined object is obtained by numerical calculations.

7. The method for examining of objects using optical devices according to claim 6, **characterized in that** the SOCT device is moved manually with respect to the slit lamp from a rest position to a neutral position and from the neutral position to an operating position, by acting with force on the elements of the SOCT device.
